# EUROPEAN PATENT APPLICATION

(11) **EP 1 470 820 A1**
(43) Date of publication of application: **27.10.2004**
(21) Application number: 02792071.9
(22) Date of filing: 27.12.2002
(51) Int. Cl.: A61K 33/44, A61K 7/00, A61K 7/48, A61K 9/08, A61K 35/72, A61K 35/74, A61K 47/32, A61K 47/36, A61K 47/38, A61P 17/00, A61P 17/02, A61P 17/10, A61P 43/00

(54) **CARBON DIOXIDE COMPOSITIONS FOR EXTERNAL USE AND PROCESS FOR PRODUCING THE SAME**

(30) Priority: 28.12.2001 JP 2001400989
(71) Applicant: Neochemir Inc., Kobe-shi, Hyogo 651-0087 (JP)
(72) Inventor: TANAKA, Masaya, Kobe-shi, Hyogo 654-0036 (JP)
(74) Representative: Sajda, Wolf E., Dipl.-Phys.
(86) International application number: PCT/JP2002/013877
(87) International publication number: WO 2003/057228

(57) **Abstract**

Carbon dioxide is dissolved in a non-bubble state in a viscous material comprising at least water and a thickener. A production method of this carbon dioxide composition for external use comprises the steps of introducing at least water and a thickener as raw materials into a carbon dioxide-impermeable sealable container, reducing the pressure inside the container to degas the raw materials, and filling carbon dioxide into the container and maintaining a sealed state, and the step of mixing the raw materials to prepare a viscous material, wherein at least during the step of filling carbon dioxide into the container. Another carbon dioxide composition for external use comprises at least a fermentation microbe, a metabolite of the fermentation microbe, a thickener, water, and carbon dioxide. With these carbon dioxide compositions for external use, strong cosmetic and medical effects are readily obtained with a small amount of the composition.

## Description

### TECHNICAL FIELD

The present invention relates to a carbon dioxide composition for external use by which cosmetic and medical effects are readily obtained and a production method of the composition.

### BACKGROUND ART

It is disclosed in Japanese Patent Application Laid-open No. 2000-319187 that transdermally or transmucosally absorbed carbon dioxide compositions that are viscous compositions containing carbon dioxide in the form of bubbles are effective against itching accompanying mucocutanecus diseases or mucocutaneous disorders such as athlete's foot, insect bites, atopic dermatitis, nummular eczema, xeroderma, seborrheic eczema, urticaria, prurigo, housewives' eczema, acne vulgaris, impetigo, folliculitis, carbuncles, furuncles, phlegmon, pyoderma, psoriasis, ichthyosis, palmoplantar keratoderma, lichen, pityriasis, wounds, burns, rhagades, erosion and chilblains; mucocutaneous injuries such as decubitus ulcers, wounds, burns, angular stomatitis, stomatitis, skin ulcers, rhagades, erosion, chilblains and gangrene; incomplete taking of skin grafts, skin flaps, etc.; dental diseases such as gingivitis, alveolar pyorrhea, denture ulcers, nigricans gingiva and stomatitis; skin ulcers, cryesthesia and numbness caused by peripheral circulatory disorders such as thromboangitis obliterans, arteriolosclerosis obliterans, diabetic peripheral circulatory disorders and lower limb varicosis; musculoskeletal diseases such as chronic rheumatoid arthritis, cervico-omo-brachial syndrome, myalgia, arthralgia and lumbago; nervous system diseases such as neuralgia, polyneuritis and subacute myelo-optic neuropathy; keratoses such as psoriasis, corns, callosity, ichthyosis, palmoplantar keratoderma, lichen and pityriasis; suppurative skin diseases such as acne vulgar is, impetigo, folliculitis, carbuncles, furuncles, phlegmon, pyoderma and suppurative eczema; suppression of regrowth of hair after depilation (disposal of unwanted hair); cosmetic problems with the skin or hair such as freckles, rough skin, loss of clarity of the skin, loss of tension or luster of the skin, and loss of glossiness of the hair; and partial obesity. However, to obtain cosmetic or medical effects using such a composition, the composition must be used in large quantities for a long time period, and the effects are weak. To obtain a face slimming effect using such a composition, for example, 26.2 g must be used everyday for one month.

A carbon dioxide packing agent for obtaining a blood circulation promoting effect of carbon dioxide is proposed in Japanese Patent Application Laid-open No. S62-286922. With this packing agent, a cloth containing water is placed onto a cloth containing a carbonate and an organic acid to generate carbon dioxide, which is dissolved in the water contained in the cloth and is thus used as dissolved carbon dioxide; however, the reaction between a carbonate and an organic acid is generally very rapid, and hence the amount of carbon dioxide diffused into the atmosphere is larger than the amount dissolved in the water, and thus cosmetic or medical effects from transdermal or transmucosal absorption of carbon dioxide cannot be expected.

In the production of carbon dioxide compositions for external use, as the method of dissolving carbon dioxide, for example, a method using a gas-liquid contactor that uses hydrophobic porous hollow fibers (Japanese Utility Model Publication No. S59-33461), a method in which carbon dioxide or a carbon dioxide-containing mixed gas and water or an aqueous solution are separated by a non-porous gas-permeable membrane, and carbon dioxide permeating through the membrane is dissolved in the water or aqueous solution (Japanese Patent Application Laid-open No. H7-779), and so on, have been proposed; however, these methods cannot be used as methods of dissolving carbon dioxide in an aqueous solution or suspension of a thickener(s), because the thickener(s) may clog up the hollow fibers or permeable membrane. Moreover, with the method in which carbon dioxide is dissolved in an aqueous solution or suspension of a thickener(s) using porous hollow fibers, the carbon dioxide is not only supplied into the aqueous layer in the form of bubbles to form a composi tion containing carbon dioxide in the form of bubbles, but also a large amount of the gas is diffused into the atmosphere; and hence the efficiency is poor.

Thus, the objective of the present invention is to provide a carbon dioxide composition for external use, which generates strong cosmetic and medical effects readily in small amount, and a production method of the composition.

### DISCLOSURE OF THE INVENTION

A carbon dioxide composition for external use of the present invention (hereinafter referred to as the 'carbon dioxide composition for external use 1') is characterized in that carbon dioxide is dissolved in a non-bubble state in a viscous material comprising at least water and a thickener (claim 1). As the results of the eager assessments by the present inventor, a composition, in which carbon dioxide is dissolved in a non-bubble state in a viscous material, was obtained and strong cosmetic and medical effects with small amount of the composition was observed, thus the present invention was accomplished. Note that 'carbon dioxide dissolved in a non-bubble state in a viscous material' in the present invention means that the viscous material does not contain bubbles of carbon dioxide, and the carbon dioxide in the viscous material is dissolved as molecular carbon dioxide at least; some of the carbon dioxide is ionized to be dissolved as carbonate ions and/or bicarbonate ions in some cases.

Another carbon dioxide composition for external use in the present invention (hereinafter referred to as 'carbon dioxide composition for external use 2') is characterized by comprising at least a fermentation microbe, a metabolite of the fermentation microbe, a thickener, water, and carbon dioxide (claim 2). Fermentation microbes produce not only carbon dioxide, but depending on the strain, possibly other bioactive substances such as various amino acids, organic acids, vitamins, enzymes and polysaccharides and so on as metabolites, and hence by selecting the type of the fermentation microbe in accordance with the purpose, a carbon dioxide composition for external use can be obtained in which a metabolite(s) give(s) additive or synergistic effects to the cosmetic or medical effects of the carbon dioxide.

In the above-mentioned two types of carbon dioxide composition for external use 1 and 2, the content of the thickener(s) is/are preferably 0.01 to 15 wt% relative to the total amount of the composition (claim 3) . With a suitable viscosity obtained by the thickener(s), the carbon dioxide composition for external use 1 or 2 in the present invention can stick to skin or mucosa suitably, and does not run down, and hence cosmetic or medical effects are obtained reliably at the site of application, and moreover the composition does not come off to soil clothing or the like.

In the above-mentioned two types of carbon dioxide composition for external use 1 and 2, the thickener(s) is/are preferably one or more selected from the group consisting of sodium alginate, propylene glycol alginate, carrageenan, sodium carboxymethyl cellulose, carboxyvinyl polymers, sodium hyaluronate, pectin, and polyvinylpyrrolidone (claim 4). Suchthickenershavehighaffinity to skin and mucosa, and moreover give good feeling in use, whereby the carbon dioxide composition for external use 1 and 2 in the present invention can be used suitably for cosmetic or medical purposes.

A production method of the carbon dioxide composition for external use 1 in the present invention is characterized by comprising the steps of introducing at least water and a thickener as raw materials into a carbon dioxide-impermeable sealable mixer and sealing the mixer, reducing the pressure inside the mixer to degas the raw materials, filling carbon dioxide into the mixer, and while the inside of the mixer is filled with carbon dioxide, mixing the raw materials, and thus preparing a viscous material having carbon dioxide dissolved therein in a non-bubble state (claim 5). Carbon dioxide is several decades of times more soluble in water than air, and hence, after the degassing, the raw materials readily absorb and dissolve carbon dioxide, whereby the carbon dioxide composition for external use 1 in the present invention can be easily produced. Even if bubbles of carbon dioxide are formed through the mixing of the raw materials or the like, if the raw materials are left for a certain period of time, then the bubbles are dissolved in the raw materials, or gather at the top, and at last completely disappear, whereby a carbon dioxide composition for external use having carbon dioxide dissolved therein in a non-bubble state can be easily produced.

Another production method of the carbon dioxide composition for external use 1 of the present invention is characterized by comprising the steps of introducing at least water and a thickener as raw materials into a carbon dioxide-impermeable sealable mixer and sealing the mixer, filling carbon dioxide into the mixer at an equal or higher pressure than atmospheric pressure, mixing the raw materials to dissolve carbon dioxide therein in a non-bubble state, and gradually reducing the pressure not to form bubbles in the raw material mixture, thus preparing a viscous material having carbon dioxide dissolved therein in a non-bubble state (claim 6). A carbon dioxide composition for external use having carbon dioxide dissolved therein in a non-bubble state can also be obtained in this way.

A production method of the carbon dioxide composition for external use 2 in the present invention is characterized by comprising the steps of introducing at least a fermentationmicrobe, a culture medium for the fermentation microbe, a thickener, and water as raw materials into a carbon dioxide-impermeable sealable container, and sealing the container (claim 7). Through respiration of the fermentation microbe, carbon dioxide is produced inside the carbon dioxide-impermeable sealable container, and due to the sealed state, carbon dioxide will not leak out to the outside at all, and hence the carbon dioxide can be utilized for the carbon dioxide composition for external use without waste; moreover, the steps of producing the composition and packing into the sealed containers, and storing can be carried out simultaneously.

In the above-mentioned carbon dioxide composition for external use 2 and its production method, the fermentation microbe is preferably yeast or lactic acid bacteria (claims 8,9). Among fermentation microbes, yeast and lactic acid bacteria are used in food industry, etc.; their microbial cells are edible; may be suitably used for cosmetic or medical purposes.

For the carbon dioxide composition for external use 1 in the present invention, carbon dioxide is dissolved in a viscous material comprising at least water and a thickener in a non-bubble state. In the viscous material, the thickener (s) may be dissolved in water, or may be suspended in water, or may be swollen by water. When carbon dioxide is dissolved in water, the volume of the carbon dioxide is virtually negligible, and hence the amount of carbon dioxide which can be included in a certain volume of a carbon dioxide-containing composition for the carbon dioxide composition for external use 1 in the present invention is larger than that of a composition containing carbon dioxide in the form of bubbles. Thus, the carbon dioxide composition for external use 1 in the present invention can provide more carbon dioxide than the same amount, i.e. the same volume, of a composition containing carbon dioxide in the form of bubbles, and hence stronger cosmetic or medical effects can be obtained with the same amount of the composition. Or, smaller amount of the composition is required to obtain the same level of cosmetic or medical effects for the carbon dioxide composition for external use 1 in the present invention than for a composition containing carbon dioxide in the form of bubbles.

The carbon dioxide composition for external use 2 in the present invention comprises at least a fermentation microbe, a metabolite of the fermentation microbe, a thickener, water, and carbon dioxide. Fermentation microbes have been utilized hitherto for agents for external use such as cosmetics and medicines, but in most cases, a metabolite(s) or the like of the fermentation microbe(s) is/are separated and purified, and then blended into the agent for external use. In the carbon dioxide composition for external use 2, the fermentation microbe (s) is/are present as live microbe(s)during production, the microbe(s) produce(s) not only carbon dioxide, but also a metabolite (s) , which is/are not separated and purified, instead to be used as it/they are formed in the composition for external use. Thus, the carbon dioxide and the metabolite(s) and so on produced can be used without wastage, and moreover, depending on the metabolite(s) and so on, additive or synergistic effects that cannot be obtained with carbon dioxide alone can also be obtained. For example, if a hyaluronic acid-producing strain of the genus Strepto such as *Streptococcus Pyogenes* is used, then the wound healing accelerating effect, viral infection prevention effect and so on of hyaluronic acid act additively or synergistically with the wound healing accelerating effect of carbon dioxide, whereby an excellent medicinal agent for external use, which can accelerate wound healing and prevent viral infection, can be provided. In the case of cosmetics, a cosmetic product with superior moisturizing effect, with the moisturizing effect of hyaluronic acid being added to the cosmetic effects of carbon dioxide, can be obtained. Or, if a dairy lactic acid bacteria strain of the genus *Strepto* such as *Streptococcus Thermophilus* is used, then moisturizing components such as lactic acid, glucose, phosphoric acid an so on are produced, and a cosmetics with an excellent moisturizing effect, with the moisturizing components acting additively or synergistically with the cosmetic effects of carbon dioxide, can be obtained. Besides these examples, by selecting the strain of the fermentation microbe as appropriate in accordance with the purpose, other carbon dioxide compositions for external use having additive or synergistic effects to the cosmetic or medical effects of carbon dioxide can be obtained. The carbon dioxide in the carbon dioxide composition for external use 2 can be present in either a bubble state or a non-bubble state.

In each of the two types of carbon dioxide composition for external use 1 and 2 in the present invention, the content of the thickener (s) is/are preferably 0.01 to 15 wt%, more preferably 0.1 to 8 wt%, and most preferably 0.5 to 4 wt%, relative to the total amount of the composition. If this content is 0.01 wt% or less, the viscosity of the composition is too low, and hence the composition runs down when applied, cosmetic or medical effects at the site of application cannot be expected, and moreover composition runs down to soil clothing or the like. If this content is 15 wt% or more, the viscosity is too high, and hence the composition does not spread well, and the application is difficult, and thus the usability is poor.

As the thickener (s) in each of the two types of carbon dioxide composition for external use 1 and 2 in the present invention, one or more selected from the group consisting of natural polymers, semi-synthetic polymers, synthetic polymers and inorganic materials, can be used.

Examples of natural polymers are polymers from plants such as gum arabic, carrageenan, galactan, agar, quince seed, guar gum, tragacanth, pectin, mannan, locust bean gum, wheat starch, rice starch, corn starch and potato starch, microbe-derived polymers such as curdlan, xanthan gum, succinoglucan, dextrin, hyaluronic acid and pullulan, and protein polymers such as albumin, casein, collagen, gelatin and fibroin; one or more of these can be used. Among these, from the standpoint of usability and so on, gum arabic, carrageenan, xanthan gum, tragacanth, hyaluronic acid, pullulan, pectin, mannan and locust bean gum can be preferably used, and from the standpoint of affinity to skin and mucosa and so on, carrageenan and pectin can be particularly preferably used.

Examples of semi-synthetic polymers are cellulosic polymers such as ethyl cellulose, carboxymethyl cellulose, carboxymethyl ethyl cellulose, carboxymethyl starch, croscarmellose, crystalline cellulose, cellulose acetate, cellulose acetate phthalate, hydroxyethyl cellulose, hydroxypropyl cellulose, hydroxypropyl methyl cellulose, hydroxypropyl methyl cellulose phthalate, methyl cellulose and methyl hydroxypropyl cellulose, starch-type polymers such as pregelatinized starch, partially pregelatinized starch, carboxymethyl starch, dextrin and methyl starch, alginate-type polymers such as sodium alginate and propylene glycol alginate, and other polysaccharide-type polymers such as chondroitin sulfate sodium and sodium hyaluronate; one or more of these can be used. Among these, from the standpoint of usability and so on, sodium alginate, propylene glycol alginate, sodium carboxymethyl cellulose, dextrin, sodium hyaluronate, hydroxyethyl cellulose, hydroxypropyl cellulose, hydroxypropyl methyl cellulose and methyl cellulose can be preferably used, and from the standpoint of affinity to skin and mucosa and so on, sodium alginate, propylene glycol alginate and sodium hyaluronate can be particularly preferably used.

Examples of synthetic polymers are carboxyvinyl polymer, sodium polyacrylate, polyvinylacetal diethylaminoacetate, polyvinyl alcohol, polyvinylpyrrolidone, methacrylic acid-ethyl acrylate copolymers, methacrylic acid-ethyl methacrylate copolymers, ethyl methacrylate-trimethylammonium chloride ethyl methacrylate copolymers, dimethylaminoethyl methacrylate-methyl methacrylate copolymers and so on; one or more of these can be used. Among these, from the standpoints of affinity to skin and mucosa, usability and soon, carboxyvinyl polymers and polyvinylpyrrolidone can be used particularly preferably.

Examples of inorganic materials are hydrated silicon dioxide, light anhydrous silica, colloidal alumina, bentonite, laponite and so on; one or more of these can be used.

The concentration of dissolved carbon dioxide in each of the two types of carbon dioxide composition for external use 1 and 2 in the present invention is preferably 60 ppm or higher, more preferably 300 ppm or higher, most preferably 1,000 ppm or higher. At lower than 60 ppm, cosmetic or medical effects are not obtained. The higher the concentration of dissolved carbon dioxide, the stronger the effects, but there is no need to increase the concentration of dissolved carbon dioxide up to the saturated level.

Dissolved carbon dioxide is readily ionized if the pH of the solution is high, whereupon the proportion of molecular carbon dioxide, which gives cosmetic or medical effects, declines; the hydrogen ion concentration of each of the two types of carbon dioxide composition for external use 1 and 2 in the present invention, is preferably at pH 2 to 9, more preferably at pH 4 to 8, and most preferably at pH 4 to 5. At pH 2 or lower, denaturation of proteins in skin, mucosa or the like may occur. At pH 9 or higher, most of the dissolved carbon dioxide is ionized, and hence the proportion of molecular carbon dioxide, which gives cosmetic and medical effects, is low. The pH of each of the two types of carbon dioxide composition for external use 1 and 2 in the present invention may fall within the range of 2 to 9 depending on types of the rawmaterials used such as a thickener(s) and so on, but in the case that the pH is out of that range, or in the case that it is necessary to adjust to a certain pH, the pH of the composition can be adjusted by using an appropriate acid(s) or base(s).

There is no particular limitation for the acid(s), and one or more selected from the group consisting of inorganic acids and organic acids commonly used in agents for external use, can be used. Examples of inorganic acids are phosphoric acid, potassium dihydrogenphosphate, sodium dihydrogenphosphate, sodium sulfite, potassium sulfite, sodium pyrosulfite, potassium pyrosulfite, acidic sodium hexametaphosphate, acidic potassium hexametaphosphate, acidic sodium pyrophosphate, acidic potassium pyrophosphate, sulfamic acid and so on; one or more of these can be used.

Examples of organic acids are straight chain fatty acids such as formic acid, acetic acid, propionic acid, butyric acid and valeric acid, dicarboxylic acids such as oxalic acid, malonic acid, succinic acid, glutaric acid, adipic acid, pimelic acid, fumaric acid, maleic acids, phthalicacid, isophthalicacidandterephthalicacid, acidic amino acids such as glutamic acid and aspartic acid, hydroxy acids such as glycolic acid, malic acid, tartaric acid, ci tric acid, lactic acid, hydroxyacrylic acid, α-oxybutyric acid, glyceric acid, tartronic acid, salicylic acid, gallic acid, tropic acid, ascorbic acid and gluconic acid, and so on; one or more of these can be used.

As the base(s), basic compounds(s) commonly used in agents for external use can be used, for example, alkali metal hydroxides such as sodium hydroxide and potassium hydroxide, alkaline earth metal hydroxides such as calcium hydroxide and magnesium hydroxide, aqueous ammonia, amines such as triethylamine, diisopropylamine, monoethanolamine, diethanolamine, triethanolamine, monoisopropanolamine, diisopropanolamine, triisopropanolamine, aminomethylpropanolamine, aminoethylpropanolamine, pyridine, piperidine and morpholine, basic amino acids such as arginine, and so on; one or more of these can be used.

In addition to the essential components previously mentioned, raw materials commonly used in agents for external use and cosmetics can be blended into the carbon dioxide composition for external use 1 in the present invention, for example, fragrances, coloring agents, surfactants, oils, moisturizing agents, alcohols, preservatives, antioxidants, metal ion chelaters, coloration preventing agents, ultraviolet absorbing/scattering agents, vitamins, amino acids, medicinal agents such as arbutin, kojic acid, nutrients, anti-inflammatories, vasodilators, hormones, astringents, antihistamines, microbicides, sebum inhibiting agents, keratin stripping/dissolving agents, anti-seborrheic agents and antipruritics, and so on, whereby the carbon dioxide composition for external use 1 can be used yet more suitably as a cosmetic or medicinal agent for external use.

In addition to the essential components previously mentioned, raw materials commonly used in agents for external use and cosmetics can be blended into the carbon dioxide composition for external use 2 in the present invention, for example fragrances, coloring agents, surfactants, oils, moisturizing agents, alcohols, antioxidants, metal ion chelaters, coloration preventing agents, ultraviolet absorbing/scattering agents, vitamins, amino acids, medicinal agents such as arbutin, kojic acid, nutrients, anti-inflammatories, vasodilators, hormones, astringents, antihistamines, sebum inhibiting agents, keratin stripping/dissolving agents, anti-seborrheic agents and antipruritics, and so on, whereby the carbon dioxide composition for external use 2 can be used yet more suitably as a cosmetic or medicinal agent for external use. Note, however, that raw materials with microbicidal, sterilizing or fungistatic/bacteriostatic effects or the like interfere the activities of the fermentation microbe, and therefore are undesirable.

With each of the two types of carbon dioxide composition for external use 1 and 2 in the present invention, cosmetic and medical effects can be obtained by simply applying the composition thinly onto skin or mucosa as an ordinary agent for external use; it is not necessary to rub it into the skin or mucosa; the composition can be applied in the way so as to just cover the skin or mucosa. The time period of application is preferably 1 minute or longer, more preferably 5 minutes or longer. At less than 1 minute, sufficient cosmetic or medical effects cannot be obtained. Longer application is preferable, but the concentration of carbon dioxide in the composition is decreased through transdermal absorption and diffusion into the atmosphere, and once the concentration has dropped to below 60 ppm, no further effect can be obtained. By using an occlusive treatment method, in which the application area of the carbon dioxide composition for external use 1 or 2 in the present invention is covered and thus closed up using a sheet, a film or the like, the cosmetic and medical effects can be enhanced. In the case of carrying out the occlusive treatment method for medical purposes, continuous application for 24 hours or longer is possible, which is convenient for treatment. There are no particular limitations on the covering material used in the occlusive treatment method so long as this material is a sheet, film or the like with low carbon dioxide permeability; for example, a synthetic polymer sheet, film or the like can be used. In the case of a medical purpose such as treating a wound in particular, an adhesive wound-covering material made of a synthetic polymer or the like can be used suitably.

Each of the two types of carbon dioxide composition for external use 1 and 2 in the present invention may be left as it is after being applied, but stickiness may remain and hence it is preferable to remove the composition. There are no particular limitations on the removal method; the composition may be wiped off with a cloth, tissue paper or the like, or can be washed off with water or the like.

Each of the two types of carbon dioxide composition for external use 1 and 2 in the present invention is preferably stored in a carbon dioxide-impermeable sealable container. Since the hydrogen ion concentration of the carbon dioxide composition for external use 1 or 2 in the present invention is preferably at pH 2 to 9, so long as the material for the container is stable physically and chemically in this pH range and is carbon dioxide-impermeable, there are no particular limitations; the material used can be selected as appropriate from polymers, metals, glasses and so on, and polymers are preferable in view of processabi lity and usability. Moreover, to be prepared for bubble formation in the carbon dioxide-impermeable sealable container when the carbon dioxide dissolved in the composition is under a high temperature environment or the like, the material of the container is preferably either highly elastic or physically strong, or both. Examples of polymers are ethylene vinyl alcohol, complex of nylon and ethylene vinyl alcohol, polyethylene, polyethylene naphtha late, polyethylene terephthalate, polycarbonates, polypropylene, polystyrene, polyvinyl chloride, polyamide of meta-xylylenediamine and adipic acid, polyacrylonitrile, vinylidene chloride, vinyl chloride-vinylidene chloride copolymers, polypropylene and so on; one or more of these can be used.

The carbon dioxide-impermeable sealable container should be sealable and from which the contents can be easily taken out; examples are a tube, a bag, a pouch, a syringe, an ampoule, a bottle or a cup with sealing cap/lid, and so on; except those mentioned here other commonly used shapes can also be used with no particular limitation. The container can be one-time use disposable type, or can be those types which can be opened to take the necessary amount and sealed completely again after opening to store the remaining carbon dioxide composition for external use by a lid, a cap, a zipper or the like; the structure can be selected as appropriate based on the way of usage.

For the carbon dioxide composition for external use 1 in the present invention, the method of dissolving carbon dioxide in the viscous liquid should be a method that carbon dioxide is dissolved without forming bubbles; moreover, the concentration of carbon dioxide dissolved can be saturated or lower than saturated, or supersaturated. For carbon dioxide composition for external use 1 in the present invention in which carbon dioxide is dissolved to the supersaturated concentration, diffusion of the dissolved carbon dioxide is suppressed by the viscosity and surface tension of the composition, depending on the type and concentration and so on of the thickener (s) used and the concentration of the dissolved carbon dioxide. And, even in the case that diffusion is not suppressed, the dissolved carbon dioxide does not diffuse completely, the diffusion stops at a certain concentration when there is no big difference in the partial pressure of the dissolved carbon dioxide and the outside pressure due to the viscosity and surface tension of the composition, carbon dioxide sufficient enough to provide cosmetic or medical effects remains in the composition, and thus there are no problems.

A preferable production method of the carbon dioxide composition for external use 1 in the present invention is characterized by comprising the steps of introducing at least water and a thickener as raw materials into a carbon dioxide-impermeable sealable mixer, reducing the pressure inside the mixer to degas the raw materials, and filling carbon dioxide into the mixer and maintaining a sealed state; and during the filling of carbon dioxide to the mixer and maintaining of the sealed state, the mixing of the rawmaterials and the dissolution or swelling of the thickener (s) are done(hereinafter referred to as 'production method 1-1'). There are no particular limitations on the method of degassing the raw materials so long as the pressure inside the mixer can be reduced below the outside pressure; a vacuum pump or the like can be used. An example of a mixer, which can be sealed and whose inside pressure can be reduced, and by which the making of thickener solution, suspension or swollen liquid is possible, is a vacuum emulsifier; a commercially available machine can be used as it is, or a custom-made equipment suited to the production method of the present invention may be manufactured. Regarding the production procedure, for example, water and a thickener (s) as essential raw materials, and other raw materials if necessary, are introduced into the carbon dioxide-impermeable sealable mixer, and the pressure inside the mixer is reduced either as it is or while mixing the raw materials to degas the raw materials; at this time, the raw materials can be stirred mechanically to promote the degassing and to mix the raw materials simultaneously, and moreover the degassing can also bepromotedbyultrasonication, vibration, or heating. Thedegassing conditions may be varied according to the types and amounts of the raw materials used and so on; during the degassing step, not only gas, but also water is reduced, and hence it is preferable to consider all of these conditions and the decrease of water during the degassing step to determine the amount of water to be introduced in production method 1-1. A higher extent of degassing is preferable, but it is not necessary to degas completely. Though it is possible to conduct filling of carbon dioxide into the mixer and degassing simultaneously, carbon dioxide is lost through the degassing, and thus there is a problem with production efficiency.

After the degassing is completed, as the next step, carbon dioxide is filled into the mixer; it is preferable to fill the carbon dioxide into the mixer while mixing the raw materials through mechanical stirring or the like so that carbon dioxide is dissolved into the whole of the raw material mixture rapidly and uniformly. At a certain liquid temperature, until the saturation level, the amount of carbon dioxide dissolved is largely dependent on the amount of carbon dioxide filled in, and hence the amount of carbon dioxide dissolved into the raw material mixture can be controlled by the amount of carbon dioxide introduced. Moreover, since the amount of carbon dioxide dissolved is larger at the lower liquid temperature, it is preferable to lower the liquid temperature to a range that the mixing of the raw materials is not inhibited.

After carbon dioxide has been dissolved in the raw material mixture and hence the carbon dioxide composition for external use 1 in the present invention is prepared, it is preferable to fill the composition into a carbon dioxide-impermeable sealable container, and seal the container. Regarding the filling, so long as the surface tension of the composition is sufficiently strong to prevent diffusion of the dissolved carbon dioxide, the composition can be transferred into a filling machine and ordinary filling can be carried out. If that is not the case, to prevent diffusion of the dissolved carbon dioxide in the composition, rather than taking the composition out from the carbon dioxide-impermeable sealable mixer used in the preparation and carrying out the filling in the outside air, it is preferable to use a closed type filling machine that is directly connected to the mixer and not influenced by the outside air, and if necessary replace the air inside the filling machine with carbon dioxide before filling, and then fill the composition into a carbon dioxide-impermeable sealable container, and immediately seal the container. The amount of the composition filled in can be the full volume of the carbon dioxide-impermeable sealable container, or can be less than that, but in the latter case, there is a space in the container, and hence it is preferable to replace the gas in that part with carbon dioxide. In the case of carrying out the filling in the outside air, if the amount of carbon dioxide lost from the carbon dioxide composition for external use 1, is within a range such as not to affect the cosmetic or medical effects of the carbon dioxide composition for external use 1, the filling can be carried out in the outside air.

In another preferable production method of the carbon dioxide composition for external use 1 in the present invention (hereinafter referred to as 'production method 1-2'), at least water and a thickener are introduced as raw materials into a carbon dioxide-impermeable sealable mixer and are mixed, and carbon dioxide is filled into the mixer at a pressure above atmospheric pressure (the outside pressure) to dissolve the required amount of carbon dioxide in a non-bubble state. Next, the pressure is gradually reduced so as not to form bubbles in the carbon dioxide composition for external use 1, thus obtaining a viscous material having carbon dioxide dissolved therein in a non-bubble state (carbon dioxide composition for external use 1), and this viscous material is immediately filled into a sealable container or the like. During the filling, to prevent diffusion of carbon dioxide from the composition, it is preferable to carry out the filling under a carbon dioxide atmosphere.

A preferable production method of the carbon dioxide composition for external use 2 in the present invention is characterized by comprising the steps of introducing at least a fermentation microbe, a culturemedium for the fermentation microbe, a thickener, and water as raw materials into a carbon dioxide-impermeable sealable container, and sealing the container (hereinafter referred to as'productionmethod 2'). For production method 2, large, expensive production equipment is not required, the production is easy, and the raw materials are relatively inexpensive. Regarding the production procedure, the above-mentioned rawmaterials, and other rawmaterials if necessary, are introduced into the carbon dioxide-impermeable sealable container, the container is sealed, and then until the required amount of carbon dioxide is produced, a temperature range within which the microbe can carry out fermentation is maintained. Note, however, as the other raw materials, raw materials that have a microbicidal, sterilizing or fungistatic/bacteriostatic effect or the like, which interfere the activity of the fermentation microbe (s) , are undesirable. Moreover, to prevent the effects from microorganisms other than the fermentation microbe, it is preferable to sterilize the raw materials except the fermentation microbe in advance.

For production method 2, the principal objective is not proliferation of the microbial cells, but production of the required amount of carbon dioxide, the temperature and time should be selected as appropriate such that the amount of carbon dioxide produced becomes high, depending on the microbial strain (s) used. Microbial strains used in the fermentation industry are generally mesophiles, and hence fermentation can be carried out at normal temperatures; if such a strain(s) is/are used, a process of heating, cooling or the like to produce and dissolve the carbon dioxide is unnecessary. In the case that the shorter production time is desired, it is possible to heat to a temperature at which carbon dioxide production becomes more active according to the strain (s) of fermentation microbe(s) used.

Regarding the producing conditions for production method 2, raw materials, fermentation temperature and fermentation time, hydrogen ion concentration and so on, under which the fermentation microbe (s) used can produce a large amount of carbon dioxide, are preferably selected for each microbial strain used. A carbon compound is essential as a carbon dioxide source for the nutrient sources in the culture medium for the fermentation microbe (s) , and regarding other nutrient sources, in some cases, a nitrogen source, an inorganic substance (s) or the like may be required, and hence the nutrient sources should be selected as appropriate in accordance with the microbial strain(s) used. Among carbon compounds, carbohydrates are preferable from the perspective of the utilization efficiency of the fermentation microbe and so on, for examples, monosaccharides such as glucose and fructose, disaccharides such as sucrose, maltose and lactose, organic acids such as citric acid, malic acid, succinic acid, fumaric acid and tartaric acid, and so on; one or more of these can be used. Among these, monosaccharides are used most easily and hence are particularly preferable. For production method 2, the proliferation of the microbial cells is not the principal objective, and hence it is generally unnecessary to add nutrient sources such as a nitrogen source or an inorganic substance(s) particularly, because, in many cases, such nutrient sources are contained as impurities in any of the raw materials.

In general, a fermentation microbe carries out fermentation consuming an easily utilized nutrient source preferentially first, and hence when all of the nutrient sources have been consumed from the culture medium for fermentation microbe, the fermentation microbe may consume other nutrient sources for fermentation. In the case that the thickener(s) is/are organic compound(s), the fermentation microbe may utilize this/these as a carbon source, whereby the thickener(s) concentration may decrease, i.e. the viscosity of the carbon dioxide composition for external use may decrease; to prevent this, for production method 2, it is preferable to suppress the activity of the fermentation microbe(s) once the required amount of carbon dioxide has been produced. Examples of methods of suppressing the activity of the fermentation microbe (s) include heating at a high temperature, irradiating with radiation, an electron beam or the like, and destroying the microbial cells by freezing and thawing the raw material mixture; a method can be selected as appropriate so that the preparation of the carbon dioxide composition for external use 2 is not affected negatively.

As the fermentation microbe(s), one or more selected from the group consisting of molds, yeasts, bacteria and actinomycetes can be used. The microbial strain used can be selected from fermentation microbes used in the fermentation industry or the like according to the purposes, or a variant which can produce a large amount of carbon dioxide can be screened from nature, or starting with an existing strain, a new strain suited to the purpose can be produced through physical treatment such as heat treatment, acid treatment or irradiation, chemical treatment using an alkylating agent such as nitrogen mustard, a base analog such as 5-bromodeoxyuridine, a pigment such as acridine orange, or another compound such as nitrous acid or hydrazine, or biotechnology such as cell fusion or gene recombination. Moreover, in accordance with the purpose, a strain that produces any of various bioactive substances such as amino acids, organic acids, vitamins, enzymes and polysaccharides and so on can be selected, or produced, whereby a carbon dioxide composition for external use 2, which has further effects additively or synergistically to the cosmetic and medical effects of the carbon dioxide, can be produced. For example, if an amino acid-producing strain belonging to any of the genera *Corynebacterium, Brevibacterium, Bacillus* and so on is used, then the moisturizing effect of amino acids is added, and if a lactic acid-producing strain of the genus *Lactobacillus* is used, then the moisturizing effect of lactic acid is added; if aerobic bacteria such as the genera *Acetobacter, Pseudomonas* or *Bacillus,* or facultative anaerobic bacteria such as lactic acid bacteria or *Escherichia coli,* is used, then an active oxygen-removing enzyme such as a catalase or a superoxide dismutase is produced, and hence aging of skin and so on can be suppressed, whereby a carbon dioxide composition for external use that can be suitably used as a cosmetic can be produced.

In production method 2, among fermentation microbes, lactic acid bacteria and yeast are particularly preferable, because there is a long history of use in foods and so on, their microbial cells are edible directly, they are active under an acidic condition in general, and so on. In general, a fermentation microbe predominantly produces carbon dioxide and water from a carbon source in an oxygen-rich environment, and hence in production method 2, it is preferable to include a step, in which at least one of the method using raw materials containing abundant oxygen, and the method introducing oxygen or air containing abundant oxygen into the carbon dioxide-impermeable sealable mixer when the raw materials are introduced before sealing, is included.

### BEST MODE FOR CARRYING OUT THE INVENTION

Followings are more concrete descriptions of the present invention with examples; however, the present invention is not limited to these examples.

### Example 1

Into a vacuum emulsifier, purified water, 50 vol% of the internal volume of the emulsifier, and taking 95.4 parts by weight, was introduced as water, 3 parts by weight of sodium alginate and 1. 5 parts by weight of sodium carboxymethyl cellulose as thickeners, and 0.1 parts by weight of methylparaben as a preservative were gradually added thereto while stirring with a rotating propeller, and then the pressure inside the emulsifier was reduced from the outside pressure (760 mmHg) to 360 mmHg after 10 minutes. Next, while continuing the stirring, carbon dioxide at the pressure adjusted to 1 kg/cm² was introduced into the emulsifier, after the pressure inside the emulsifier was recovered to the outside pressure, and then a gas exhausting valve of the emulsifier was opened to discharge the gas to the outside, and carbon dioxide continued to be introduced in for further 5 minutes, thus replacing the gas inside the emulsifier with carbon dioxide. The gas exhausting valve was then closed, the introduction of carbon dioxide was suspended, thus leaving the vacuum emulsifier into a sealed state, and then while continuing the stirring, upon the pressure inside the emulsifier falling below the outside pressure, carbon dioxide was introduced so that the pressure inside the emulsifier recovered to the outside pressure. The stirring was carried out for 3 hours with the pressure inside the emulsifier at a constant value, which is equal to the outside pressure, and then the system was left as it is in a sealed state for 1 day, whereby a carbon dioxide composition for external use 1 according to production method 1-1 was prepared.

### Example 2

95 parts by weight of purified water as water, 3 parts by weight of propylene glycol alginate and 1 part by weight of sodium carboxymethyl cellulose as thickeners, and 0.5 parts by weight of glucose as a culture medium for fermentation microbe were mixed together in a polypropylene tube to prepare a viscous composition, and then 0.5 parts by weight of yeast was added as a fermentation microbe and stirred, and the tube was immediately sealed with a sealing cap. The sealed tube was left standing at room temperature for 1 week, and then it was sterilized by heating at 120°C in an autoclave for 20 minutes, whereby a carbon dioxide composition for external use 2 according to production method 2 was prepared.

### Example 3

94 parts by weight of purified water as water, 4 parts by weight of sodium alginate as a thickener, 1 part by weight of yeast as a fermentation microbe, and 1 part by weight of glucose as a culture medium for the yeast were mixed together in a polypropylene tube, and the tube was immediately sealed with a sealing cap. The sealed tube was left standing at room temperature for 2 days, and was then immersed in hot water at 80°C for 20 minutes, whereby a carbon dioxide composition for external use 2 according to production method 2 was prepared.

### Example 4

94 parts by weight of purified water as water, 4 parts by weight of sodium alginate as a thickener, and 1 part by weight of glucose as a culture medium for fermentation microbe were mixed together in a polypropylene tube to prepare a viscous composition, and then 1 part by weight of yeast as a fermentation microbe was further mixed in, and the tube was immediately sealed with a sealing cap. The sealed tube was left standing at 40°C for 4 hours, and was then immersed in hot water at 80°C for 20 minutes, whereby a carbon dioxide composition for external use 2 according to production method 2 was prepared.

### Example 5

98.2 parts by weight of purified water as water, 1 part by weight of carrageenan as a thickener, 0.4 parts by weight of yeast as a fermentation microbe, and 0.4 parts by weight of glucose as a culture medium for the yeast were mixed together in a polypropylene tube, and the tube was immediately sealed with a sealing cap. The sealed tube was left standing at room temperature for 2 days, and was then immersed in hot water at 80°C for 20 minutes, whereby a carbon dioxide composition for external use 2 according to production method 2 was prepared.

### Example 6

93 parts by weight of purified water as water, 5 parts by weight of a carboxyvinyl polymer as a thickener, and 1 part by weight of glucose as a fermentation microbe culture medium were mixed together in a polypropylene tube to prepare a viscous composition, and then 1 part by weight of yeast as a fermentation microbe was further mixed in, and the tube was immediately sealed with a sealing cap. The sealed tube was left standing at 40°C for 3 hours, and was then immersed in hot water at 80°C for 20 minutes, whereby a carbon dioxide composition for external use 2 according to production method 2 was prepared.

### Example 7

94 parts by weight of purified water as water, 4 parts by weight of pectin as a thickener, 1 part by weight of yeast as a fermentation microbe, and 1 part by weight of glucose as a culture medium for the yeast were mixed together in a polypropylene tube, and the tube was immediately sealed with a sealing cap. The sealed tube was left standing at 40°C for 1 hour, and was then immersed in hot water at 80°C for 20 minutes, whereby a carbon dioxide composition for external use 2 according to production method 2 was prepared.

### Example 8

Into a vacuum emulsifier, purified water, 50 vol% of the internal volume of the emulsifier and taking 95.4 parts by weight, was introduced as water, 3 parts by weight of sodium alginate and 1. 5 parts by weight of sodium carboxymethyl cellulose as thickeners, and 0.1 parts by weight of methylparaben as a preservative were gradually added thereto while stirring with a rotating propeller, and then the pressure inside the emulsifier was increased from atmosphere pressure (760 mmHg) to 1100 mmHg, carbon dioxide was introduced and dissolved in a non-bubble state. After that, the pressure was reduced to atmosphere pressure gradually so that bubbles were not formed in the raw material mixture. After that, the mixture was immediately filled into a sealed container, whereby a carbon dioxide composition for external use 1 according to production method 1-2 was prepared.

### Comparative Example

An agent for external use containing carbon dioxide in the form of bubbles (hereinafter referred to as the 'agent for external use of the Comparative Example') was produced as follows in accordance with Example 109 in Japanese Patent Application Laid-open No. 2000-319187, that is one of the compositions to produce carbon dioxide through the reaction between a carbonate and an acid.

### Production of acid-containing material

Using 25 wt% of citric acid, 25 wt% of ethyl cellulose, and 50 wt% of croscarmellose sodium, porous columnar granules with length of approximately 4 mm and diameter of approximately 1 mm were produced through a wet extruding granulation method.

### Production of carbonate-containing composition

2.4 wt% of sodium bicarbonate was dissolved in 89.6 wt% of purified water, and while heating gradually to 60°C, 4.0 wt% of sodium alginate, 2.0 wt% of ethyl cellulose and 2.0 wt% of sodium carboxymethyl cellulose were gradually added and dissolved while stirring, and then the solution was left overnight, thus cooled the solution down to room temperature and hence a viscous composition was produced.

1 part by weight of the above-mentioned acid-containing material and 20 parts by weight of the above-mentioned viscous composition were mixed to prepare a carbon dioxide agent for external use containing carbon dioxide in the form of bubbles. Evaluation tests

The carbon dioxide compositions for external use produced as described above were evaluated as follows.

### Evaluation 1: Whitening and skin beautifying effects for hands

For seven female panelists aged 21 to 41, 5 g of one of the carbon dioxide compositions for external use of Examples 1 to 8 was applied thinly over the whole of the back of the left hand, and 5 g of the agent for external use of the Comparative Example was applied thinly over the whole of the back of the right hand. For all of the panelists, the back of the left hand, on which one of the carbon dioxide compositions for external use of Examples 1 to 8 had been applied, immediately turned red, and hence an increase in blood flow was observed, but such an increase in blood flow was not observed for the back of the right hand, on which the agent for external use of the Comparative Example had been applied. After 5 minutes when each composition was completely removed, for all of the panelists, the skin on the back of the left hand, on which one of the carbon dioxide compositions for external use of Examples 1 to 8 had been applied, was whiter, appeared more transparent, felt more moisturized, and was smoother, compared with the skin before the application, and compared with the skin on the back of the right hand. The effects of the carbon dioxide composition for external use of Example 4 were particularly strong. For the back of the right hand on which the agent for external use of the Comparative Example had been applied, the cosmetic effects such as mentioned above compared with the skin before the application were not observed for any of the panelists.

### Evaluation 2: Partial slimming, whitening and skin beautifying effects for face

For seven female panelists aged 21 to 41, 10 g of the carbon dioxide composition for external use of Example 4 was applied thinly onto the right half of the face, and 10 g of the carbon dioxide agent for external use containing carbon dioxide in the form of bubbles of the Comparative Example was applied thinly onto the left half of the face. For all of the panelists, the right half of the face, on which the carbon dioxide composition for external use of Example 4 had been applied, immediately turned red, and hence an increase in blood flow was observed, but such an increase in blood flow was not observed for the left half of the face, on which the agent for external use of the Comparative Example had been applied. After 7 minutes when each composition was completely removed, for all of the panelists, the skin on the right half of the face, on which the carbon dioxide composition for external use of Example 4 had been applied, was whiter, appeared more transparent, felt more moisturized, and was smoother, compared with the left half of the face, and moreover the corners of the mouth were raised, and the cheeks had become smaller. For the left half of the face on which the agent for external use of the Comparative Example had been applied, the effects such as mentioned above compared with the skin before the application were not observed for any of the panelists.

### Evaluation 3: Effect on abrasion

5 g of the carbon dioxide composition for external use of Example 1 was applied onto an abrasion (approximately 4 cm × 7 cm) on the right thigh of a 12-year-old boy, and a wound-coveringmaterial (trade name Tegaderm, made by 3M Health Care) was used to cover the area. This treatment was carried out once per day, and on the second day, bleeding had completely stopped, and on the fifth day, the wound had healed with no scar left.

### Evaluation 4: Effect on atopic dermatitis

10 g of the carbon dioxide composition for external use of Example 3 was applied once per day for 10 minutes on the scabs of atopic dermatitis accompanied by bleeding on the backs of both hands of a 29-year-old male, whereupon on the twelfth day, the blue-black colored skin over the whole area recovered to almost normal color, although there were still scabs. After that, the application was continued twice per week, and after two months, the scabs had been disappeared and the skin color had become completely normal.

### Evaluation 5: Treatment of acnes

5 g of the carbon dioxide composition for external use of Example 5 was applied once per day for 10 minutes on a large number of acnes accompanied by redness on the forehead of a 17-year-old male, whereupon after the first day of application, the redness had lessened and there had been an improvement in the roughness of the whole skin, and after 6 days, the acnes had been disappeared completely and the skin had become smooth and white.

### INDUSTRIAL APPLICABILITY

As described above, by the carbon dioxide compositions for external use in the present invention, strong cosmetic and medical effects can be obtained by simply applying a small amount of the compositions thinly for a short time period. According to a production method of the carbon dioxide compositions for external use in the present invention, using a carbon dioxide-impermeable sealable mixer, a carbon dioxide composition for external use in the present invention, in which carbon dioxide is dissolved in a non-bubble state in a viscous material, can be manufactured easily in large scale. According to the other production method of a carbon dioxide composition for external use in the present invention, a fermentation microbe(s) is/are used to produce carbon dioxide in a sealed container, whereby the production of the carbon dioxide composition for external use and the filling of the carbon dioxide composition for external use into a sealed container can be carried out at the same time, and moreover depending on the microbial strain(s), secondary metabolites that act additively or synergistically with the cosmetic and medical effects of the carbon dioxide can be produced, whereby a carbon dioxide composition for external use having yet higher added value is obtained. Thus, such carbon dioxide compositions for external use can be suitably used as cosmetics or medicinal agents for external use or the like.

## Claims

1. A carbon dioxide composition for external use, **characterized in that** carbon dioxide is dissolved in a non-bubble state in a viscous material comprising at least water and a thickener.

2. A carbon dioxide composition for external use, **characterized by** comprising at least a fermentation microbe, a metabolite of the said fermentation microbe, a thickener, water, and carbon dioxide.

3. The carbon dioxide composition for external use according to claim 1 or 2, **characterized by** containing 0.01 to 15 wt% of the said thickener(s) relative to the total amount of the composition.

4. The carbon dioxide composition for external use according to any of claims 1 through 3, **characterized in that** the said thickener(s) is/are one or more selected from the group consisting of sodium alginate, propylene glycol alginate, carrageenan, sodium carboxymethyl cellulose, carboxyvinyl polymers, sodium hyaluronate, pectin, and polyvinylpyrrolidone.

5. A production method of a carbon dioxide composition for external use, **characterized by** comprising the steps of introducing at least water and a thickener as raw materials into a carbon dioxide-impermeable sealable mixer and sealing the mixer, reducing the pressure inside the mixer to degas the raw materials, filling carbon dioxide into the mixer, and in a state in which the inside of the mixer is filled with carbon dioxide, mixing the raw materials, thus preparing a viscous material having carbon dioxide dissolved therein in a non-bubble state.

6. A production method of a carbon dioxide composition for external use, **characterized by** comprising the steps of introducing at least water and a thickener as raw materials into a carbon dioxide-impermeable sealable mixer and sealing the mixer, filling carbon dioxide into the mixer at a pressure equal to or higher than atmospheric pressure, mixing the raw materials to dissolve carbon dioxide therein in a non-bubble state, and gradually reducing the pressure not to formbubbles in the raw material mixture, thus preparing a viscous material having carbon dioxide dissolved therein in a non-bubble state.

7. A production method of a carbon dioxide composition for external use, **characterized by** comprising the steps of introducing at least a fermentation microbe, a culture medium for the fermentation microbe, a thickener, and water as raw materials into a carbon dioxide-impermeable sealable container, and sealing the container.

8. The carbon dioxide composition for external use according to claim 2, **characterized in that** said fermentation microbe is yeast or lactic acid bacteria.

9. The production method of a carbon dioxide composition for external use according to claim 7, **characterized in that** the said fermentation microbe is yeast or lactic acid bacteria.
